# FASCICULE DE BREVET EUROPEEN

(11) **EP 0 521 761 B1**
(45) Date de publication et mention de la délivrance du brevet: **23.10.1996**
(21) Numéro de dépôt: 92401835.1
(22) Date de dépôt: 29.06.1992
(51) Int. Cl.: A61L 15/30, A61L 15/44

(54) **Pansement cicatrisant**
Heilender Wundverband
Healing wound dressing

(30) Priorité: 03.07.1991 FR 9108305
(43) Date de publication de la demande: 07.01.1993
(73) Titulaire: LABORATOIRES D'HYGIENE ET DE DIETETIQUE L.H.D. Société Anonyme dite:, 75008 Paris (FR)
(72) Inventeur: Guillemet, Alain, F-21121 Fontaine lès Dijon (FR); Janod, Philippe, F-39100 Dole (FR)
(74) Mandataire: Clisci, Serge

(56) Documents cités:
- EP-A- 0 130 061
- EP-A- 1 004 005
- GB-A- 2 089 351
- US-A- 4 386 179
- WORLD PATENTS INDEX LATEST, accession no. 91-256236, semaine 35, Derwent Publications Ltd, Londres, GB; & JP-A-3 166 280
- WORLD PATENTS INDEX LATEST, accession no. 90-256395, semaine 34, Derwent Publications Ltd, Londres, GB; & JP-A-2 178 206
- WORLD PATENTS INDEX LATEST, accession no. 86-294595, semaine 45, Derwent Publications Ltd, Londres, GB; & JP-A-61 215 682
- PATENT ABSTRACTS OF JAPAN, vol. 4, no. 2 (C-69), 9 janvier 1980; & JP-A-54 138 124
- Handbook of Pressure Sensitive Adhesive Technology, 2nd ed., 1989, VNR New York, pages 317-331

## Description

La présente invention concerne un nouveau pansement cicatrisant comprenant un mélange de copolymères blocs à séquence centrale saturée et un plastifiant, defini en détail dans ce qui sind.

Les principales familles de pansements sont les gazes sèches, les tulles gras, les films occlusifs et les pansements occlusifs absorbants. Tous ces pansements ont pour fonction première de couvrir et protéger la plaie de l'environnement extérieur, en particulier des agressions mécanique, thermique, chimique et bactérienne.

Les gazes sèches possèdent, en plus des propriétés précédemment citées, une fonction d'absorption des exsudats. Un inconvénient majeur de ce type de pansement consiste en la formation d'un granulome à corps étranger provoqué par la libération dans la plaie de micro-fragments de la trame en coton, générant une réponse inflammatoire importante. Un autre inconvénient consiste dans l'assèchement de la plaie qui a pour conséquence, à l'ablation du pansement, de détruire les tissus néoformés provoquant ainsi un retard à la cicatrisation.

Pour diminuer l'adhérence de la gaze sèche on a proposé de l'enduire d'un corps gras. Les tulles gras sont ainsi constitués d'une gaze sèche enduite d'huiles minérales, notamment la vaseline. Cependant, au contact de la plaie, la vaseline se ramollit en raison de l'élévation de température et tend à être repoussée en périphérie du pansement. Ce phénomène est amplifié en présence d'exsudats et laisse les fibres textiles du tulle au contact direct de la plaie comme dans le cas de la gaze sèche. La cicatrisation se déroule alors dans un environnement semi-occlusif dont les principales conséquences directes sont la diminution de l'évaporation des exsudats, l'augmentation de l'humidité et de la température locale, la favorisation de la détersion, l'absence de formation de croûte, la stimulation de la granulation et la favorisation de la multiplication microbienne. On observe finalement un retard à la cicatrisation sous tulle gras.

L'occlusivité ayant une action favorable sur la cicatrisation, on a proposé des films occlusifs comme par exemple le pansement OPSITE^{R} constitué d'un film de polyuréthanne adhésif. A ce sujet, on pourra se référer de façon utile au brevet FR-B-2 012 584.
L'inconvénient essentiel d'un tel pansement est son incapacité à absorber les exsudats provoquant ainsi un risque de macération de la plaie, de rupture du pansement et de développement d'une flore bactérienne pathogène.

On a également proposé des pansements occlusifs absorbants, en particulier les pansements hydrocolloïdes, essentiellement constitués de carboxyméthylcellulose, de pectine et de gélatine. A ce sujet, on pourra se référer utilement aux brevets US-A-3 339 546 et FR-B-2495 473. De par sa composition, la masse adhésive de tels pansements se transforme en gel doux et humide au contact de la plaie développant ainsi un micro-environnement favorable à la multiplication cellulaire, à la synthèse de collagène, à la fibrinolyse, à l'angiogénèse et au bon déroulement du bactériocycle de la plaie. Malgré ce micro-climat favorable les résultats des études de cicatrisation sur ce type de pansement restent partagés. La cicatrisation n'est pas toujours accélérée de façon significative.

On connait enfin de la demande de brevet japonais JP-A-54 138 124 un pansement adhérant bien à la peau, mais facile à enlever, dont la matrice est à base d'un mélange de copolymères blocs non vulcanisés du type A-B-A, notamment les poly(styrène-butadiène-styrène) et poly(styrène-isoprène-styrène) dissous dans une substance huileuse.
Ces copolymères diffèrent des copolymères utilisés dans le cadre de la présente invention par la structure chimique de la séquence élastomérique centrale qui est insaturée pour les copolymères préconisés par JP-A-54 138 124 alors que la présente invention préconise des copolymères dont la séquence centrale est saturée. De plus le pansement selon JP-A-54 138 124 possède un pouvoir cicatrisant nettement inférieur au pansement cicatrisant selon l'invention comme il est montré dans les essais comparatifs reproduits dans la présente description.

On propose à présent une nouvelle solution technique telle que le pansement cicatrisant selon l'invention protège la plaie de l'environnement extérieur et retient les exsudats en créant ainsi un milieu humide favorable à la croissance et à la multiplication cellulaire et est non adhérent à la plaie ce qui a pour conséquence d'éviter le traumatisme de la plaie lors de l'enlèvement du pansement et de favoriser l'épithélialisation dans de bonnes conditions.

Le pansement à matrice contenant un copolymère blocs A-B-A selon l'invention est caractérisé en ce qu'il comprend dans sa matrice 10 à 30 parties en poids d'un copolymère blocs où le bloc terminal A est du type polystyrène et le bloc central B est du type polyoléfine saturée, notamment le polystyrène-polyéthylène-butylène-polystyrène et 70 à 90 parties en poids de plastifiant, notamment la vaseline.
La masse constituée par le mélange de copolymères blocs à séquence centrale saturée et de plastifiant est non adhésive.

On exclut donc de la présente invention les copolymères blocs à séquence centrale insaturée composés de blocs terminaux polystyrène et d'une séquence centrale constituée d'un bloc polyoléfinique insaturé, comme par exemple les copolymères polystyrène-polybutadiènepolystyrène ou polystyrène-polyisoprène-polystyrène.

Avantageusement on préférera les copolymères polystyrène-polyéthylène-butylène-polystyrène (en abrégé S-EB-S) notamment les S-EB-S dont le rapport nombre de motifs styrène/nombre de motifs éthylènebutylène est inférieur à 0,5 et de préférence de l'ordre de 0,4. Ces copolymères sont en particulier commercialisés par la firme Shell Chemical Company sous la dénomination Kraton^{R} G.

Par plastifiant on entend ici un corps gras, liquide ou pâteux, notamment les huiles minérales, les paraffines liquides ou pâteuses, les huiles ou graisses de silicones. On préférera les plastifiants ayant un point de goutte inférieur à 70°C et plus particulièrement les vaselines dont le point de goutte est compris entre 40 et 60°C.

Avantageusement, le pansement cicatrisant selon l'invention est caractérisé en ce qu'il est constitué d'une masse non adhésive comprenant 15 à 25 parties en poids de polystyrène-polyéthylène-butylène-polystyrène dont le rapport motifs styrène/motifs éthylène-butylène est inférieur à 0,5 et de préférence de l'ordre de 0,4 et 75 à 85 parties en poids de vaseline dont le point de goutte est compris entre 40 et 60°C.

Bien que cela ne soit généralement pas nécessaire, le mélange de copolymère blocs à séquence centrale saturée et de plastifiant selon l'invention étant une masse ayant une cohésion suffisante, on pourra ajouter une trame noyée dans la masse résultant du mélange de copolymère blocs à séquence centrale saturée et de plastifiant. Une telle trame peut être constituée de fibres naturelles ou non comme par exemple les fibres de coton ou de polyamide tissées ou non-tissées. Il est important que la trame soit totalement noyée dans la masse constituant le pansement de façon à exclure toute possibilité d'adhérence des fibres à la plaie.

Si nécessaire, on pourra recouvrir le pansement cicatrisant selon l'invention avec un support, comme par exemple les films de polyuréthanne ou de polyester ou un tissu ou un non-tissé constitué de fibres naturelles ou synthétiques. Un tel support est destiné à protéger le pansement selon l'invention, le pansement devant alors être appliqué sur la plaie ou la peau le support étant à l'opposé de la plaie ou la peau.

On pourra encore additionner à la masse constituée du copolymère blocs à séquence centrale saturée et du plastifiant un principe actif présent en quantité thérapeutiquement efficace. Ledit principe actif sera additionné à ladite masse soit en fusion si sa température de fusion est compatible avec la température de mélange des ingrédients constitutifs de la masse, soit solide à une température compatible avec la température de mélange des ingrédients constitutifs de la masse, soit encore en solution dans le plastifiant.
A titre d'exemple de principes actifs on citera les anti-inflammatoires stéroïdiens ou non, les antalgiques, les antibiotiques, les antibactériens, les antifongiques, les antiseptiques, les anesthésiques de surface, les enzymes protéolytiques, les adjuvants de la cicatrisation, les facteurs de croissance et la propolis.
Afin d'accélérer la libération du principe actif on pourra, si nécessaire, ajouter un agent promoteur du relargage du principe actif. A cet effet, on préconise notamment les polyols et les esters des alkylèneglycols, comme par exemple l'éther monoéthylique du diéthylèneglycol ou le dipropylèneglycol. La quantité d'agent promoteur du relargage du principe actif sera comprise entre 0,1 et 5 %, de façon avantageuse de l'ordre de 2 à 2,5 %, par rapport au poids total du pansement.

Le procédé de préparation d'un pansement selon l'invention consiste à mélanger dans un malaxeur le copolymère blocs à séquence centrale saturée, le plastifiant et éventuellement le principe actif et l'agent promoteur du relargage du principe actif à la température de ramollissement du copolymère, malaxer le mélange obtenu pendant environ 1 heure, puis, à une température inférieure de 10 à 40°C à la température de ramollissement du copolymère, enduire le mélange sur un support siliconé, pelable avant emploi, de façon à obtenir une épaisseur comprise entre 10 µm et 1 cm, de préférence 100 µm à 500 µm, laisser refroidir et enfin découper le produit ainsi obtenu à la dimension désirée.
Si nécessaire, l'étape d'enduction sera réalisée de façon à ce qu'une trame soit noyée dans la masse constituée par le copolymère blocs à séquence centrale saturée et le plastifiant.

Le pansement cicatrisant ainsi obtenu pourra être conditionné dans une pochette protectrice, par exemple une pochette thermosoudée en polyester téréphtalate/polyéthylène entre deux protecteurs en polyester siliconé.
Si nécessaire, le pansement cicatrisant sera stérilisé par irradiation aux rayons gamma à une dose comprise entre 25 et 32 kGy.

L'invention sera mieux comprise à la lecture des exemples de préparation et des essais pharmacologiques qui suivent.

### PREPARATION I (exemple 1)

Dans un malaxeur chauffé à 130°C on introduit 42,5 kg de vaseline officinale commercialisée sous la marque FINA^{R} F 7850 de point de goutte égal à 57°C et 15 kg de copolymère polystyrène-polyéthylène-butylène-polystyrène commercialisé par la Société SHELL sous la marque KRATON^{R} G 1652 (rapport nombre de motifs styrène/nombre de motifs éthylène-butylène de 29/71 soit 0,40). On homogénéise l'ensemble pendant 1 heure puis on incorpore à nouveau 42,5 kg de vaseline officinale FINA^{R} F 7850 et on homogénéise à nouveau l'ensemble pendant 0,5 heure. Le mélange obtenu, fluide et transparent, est enduit sur un film de polyester siliconé d'épaisseur 40 µm, à la température de 105°C de façon à obtenir une épaisseur de 400 µm. Le film ainsi obtenu est découpé en pansements rectangulaires de 3cm x 6cm et chaque pansement est conditionné dans une enveloppe thermosoudée en polyester téréphtalate/polyéthylène puis radio-stérilisé par irradiation gamma à la dose de 25 kGy.

### PREPARATION II (exemple 2)

On opère de façon analogue à la préparation I en introduisant dans le malaxeur 37,5 kg de vaseline officinale FINA^{R} F 7850 et 25 kg de copolymère polystyrène-polyéthylène-butylène-polystyrène KRATON^{R} G 1652 puis à nouveau 37,5 kg de vaseline officinale FINA^{R} F 7850.

### PREPARATION III (exemple 3)

On opère de façon analogue à la préparation I en remplaçant la vaseline FINA^{R} F 7850 par la même quantité de vaseline PROLABO RECTAPUR^{R} de point de goutte compris entre 50 et 55°C commercialisée par la Société RHONE-POULENC.

### PREPARATION IV (exemple 4)

On opère de façon analogue à la préparation I en remplaçant le copolymère polystyrène-polyéthylène-butylène-polystyrène KRATON^{R} G 1652 par un copolymère polystyrène-polyéthylène-butylène-polystyrène KRATON^{R} G 1650 (rapport nombre de motifs styrène/nombre de motifs éthylène-butylène de 0,40).

### PREPARATION V (exemple 5 comparatif )

Dans un malaxeur chauffé à 150°C on introduit 25 kg d'huile de paraffine commercialisée sous la marque SIDEPALINE^{R} BC 015 puis 5 kg de copolymère polystyrène-polybutadiène-polystyrène commercialisé par la Société SHELL sous la marque Cariflex^{R} TR 1101 et 15 kg de copolymère polystyrène-polyisoprène-polystyrène commercialisé sous la marque Cariflex^{R} TR 1107. On homogénéise l'ensemble pendant 1 heure puis on incorpore à nouveau 50 kg d'huile de paraffine et on homogénéise à nouveau l'ensemble pendant 0,5 heure. Le mélange obtenu est enduit, de façon à obtenir une épaisseur de 1 mm, sur un film de polyéthylène siliconé de 40 µm d'épaisseur, à la température de 125°C puis contre-collé par un non-tissé constitué de 55g/m² de fibres de polyester, commercialisé sous la marque SONTARA^{R} par la Société Dupont de Nemours. Le pansement obtenu est ensuite conditionné de la même façon que le pansement de l'exemple 1.

### PREPARATION VI (exemple 6)

On chauffe au bain-marie, pendant 1 heure à 90°C, 150 g du produit obtenu à l'exemple 1. On additionne ensuite 50 g d'un pré-mélange obtenu en mélangeant à froid 250 g de vaseline officinale FINA^{R} F 7850 et 250 g de sel de sodium de rifamycine. Après 5 minutes d'agitation le mélange obtenu est coulé dans une barquette en polyester siliconé. Après refroidissement, le produit solide obtenu est extrait de la barquette et pressé à 90°C pendant 2 minutes pour obtenir une plaque de 350 µm d'épaisseur que l'on découpe en pansements de dimension 10 cm x 10 cm.

### PREPARATIONS VII à XIV (examples 7 à 14)

En opérant de façon analogue à la préparation VI on a obtenu les produits suivants :
- **exemple 7 :** on a remplacé la rifamycine par la même quantité de sulfate de néomycine.
- **exemple 8 :** on a remplacé la rifamycine par la même quantité d'ibuprofène.
- **exemple 9 :** on a remplacé la rifamycine par la même quantité de benzoate de sodium.
- **exemple 10 :** le pré-mélange est constitué de 115 g de vaseline officinale FINA^{R} F 7850 et 115 g de chlorure de benzalkonium.
- **exemple 11 :** le pré-mélange est constitué de 100 g de vaseline officinale FINA^{R} F 7850 et 100 g de chlorhydrate de lidocaïne.
- **exemple 12 :** le pré-mélange est constitué de 100 g de vaseline officinale FINAR F 7850 et 100 g de benzoate de sodium.
- **exemple 13 :** le pré-mélange est constitué de 95 g de vaseline officinale FINA^{R} F 7850 et 5 g de triamcinolone.
- **exemple 14 :** le pré-mélange est constitué de 150 g de vaseline officinale FINA^{R} F 7850 et 50 g de nitrate d'éconazole.

L'activité microbiologique des produits des exemples 6 et 7 a été vérifiée in vitro. Les produits présentent une activité anti-bactérienne conforme à la Pharmacopée européenne.

Le pansement selon l'invention possède des propriétés cicatrisantes particulièrement remarquables.
Ces propriétés cicatrisantes ont été étudiées sur un modèle de cicatrisation non retardée chez l'animal en comparaison avec le produit de l'exemple 5 constitué de copolymères à séquence centrale insaturée et en comparaison avec une gaze sèche 100% coton et un tulle gras commercialisé sous la marque Tulle Gras Lumière^{R}.

Chaque lot est constitué de 10 rats non consanguins WISTAR. Sur chaque animal on pratique une plaie de 10 cm² (2cm x 5cm) paravertébrale sur un flanc de l'animal. La plaie est réalisée en conditions aseptiques par excision dermo-épidermique incluant le muscle peaucier. Immédiatement après excision un pansement (3cm x 6cm) à étudier est appliqué sur la plaie de façon à la recouvrir totalement. Le pansement est recouvert d'une gaze stérile et l'ensemble est maintenu à l'aide d'une bande adhésive. Le pansement est enlevé tous les 3 jours, la plaie est nettoyée par rinçage avec une solution stérile de chlorure de sodium et on applique ensuite un nouveau pansement jusqu'à obtenir une cicatrisation totale. Le jour de l'excision, puis tous les 3 jours jusqu'à cicatrisation totale, l'aspect macroscopique de la plaie est noté, la plaie est photographiée dans des conditions standardisées et on analyse comparativement et quantitativement (périmètre et surface) l'évolution de la plaie.

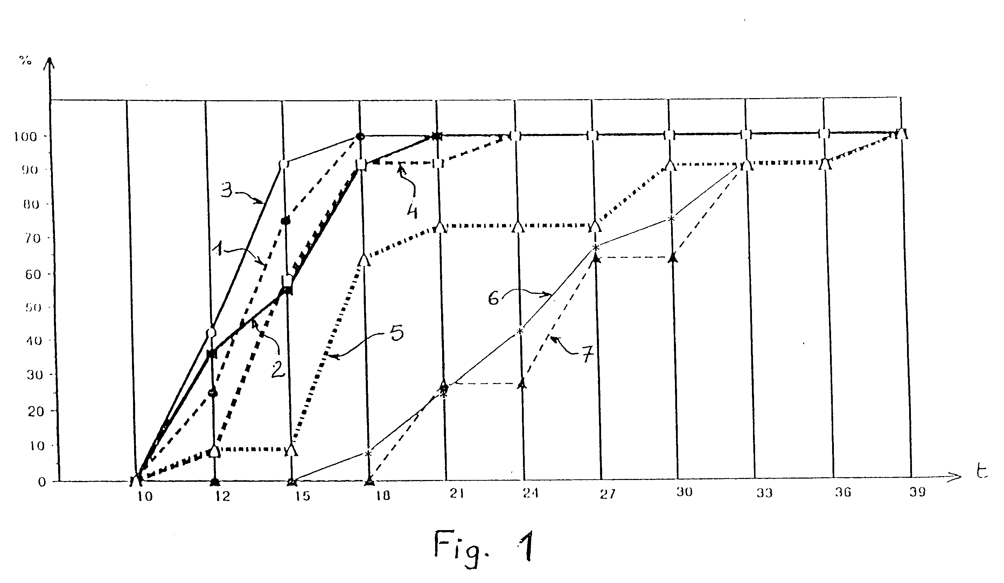

Les résultats obtenus sont présentés sur la figure 1 dans le système pourcentages (%) d'animaux cicatrisés en ordonnées en fonction du temps (exprimé en jour) en abscisses. La courbe 6 est celle obtenue avec la gaze sèche, la courbe 7 celle obtenue avec le tulle gras Lumière^{R}, la courbe 3 celle obtenue avec le pansement de l'exemple 3, la courbe 1 celle obtenue avec le pansement de l'exemple 1, la courbe 2 celle obtenue avec le pansement de l'exemple 2, la courbe 4 celle obtenue avec le pansement de l'exemple 4, la courbe 5 celle obtenue avec l'exemple 5 comparatif.
On a représenté le pourcentage d'animaux totalement cicatrisés en fonction du nombre de jours de cicatrisation. Les pansements selon l'invention permettent d'obtenir une cicatrisation totale, dès le 18ème jour pour les pansements des exemples 1 et 3, le 21ème jour pour le pansement de l'exemple 2 et le 24ème jour pour le pansement de l'exemple 4. Les pansements constitués de gaze sèche, de tulle gras ou de copolymères à séquence centrale insaturée ne permettent d'obtenir la cicatrisation totale qu'au 39ème jour. Les pansements selon l'invention présentent donc un pouvoir cicatrisant nettement supérieur aux pansements connus de l'art antérieur.

De plus, on a observé que chez les animaux traités par la gaze sèche et le tulle gras, la surface de la plaie augmente jusqu'au 3ème jour pour représenter près de 120 % de la surface de la plaie initiale. Chez les animaux traités par le pansement comparatif de l'exemple 5 la plaie ne varie pas de surface jusqu'au 3ème jour. Par contre, chez les animaux traités par les pansements des exemples 1 à 4 selon l'invention, la surface de la plaie diminue dès le 1er jour pour ne représenter plus que 80 % de la surface initiale au 3ème jour. Ceci représente un avantage considérable par rapport aux pansements connus de l'art antérieur, notamment dans le traitement des brûlures où il est important de conserver la surface de peau non brûlée et d'augmenter rapidement la surface de peau saine.

Les pansements selon l'invention sont utiles dans le soin et le traitement des plaies et brûlures en médecine humaine et vétérinaire ainsi qu'en chirurgie. Ils sont également utiles dans le traitement des affections cutanées comme par exemple les psoriasis, eczémas et dermatoses et des plaies d'origine dermatologique.

## Revendications (Revendications pour l'(les) Etat(s) contractant(s) suivant(s): AT, BE, CH, DE, DK, FR, GB, IT, LI, LU, MC, NL, PT, SE)

1. Pansement à matrice contenant un copolymère blocs A-B-A, caractérisé en ce qu'il comprend dans sa matrice 10 à 30 parties en poids d'un copolymère blocs où le bloc terminal A est du type polystyrène et le bloc central B est du type polyoléfine saturée, et 70 à 90 parties en poids de plastifiant.

2. Pansement selon la revendication 1, caractérisé en ce que sa matrice est constituée de 10 à 30 parties en poids dudit copolymère blocs A-B-A et de 90 à 70 parties en poids dudit plastifiant pour 100 parties en poids de ladite matrice.

3. Pansement selon la revendication 1 ou 2, caractérisé en ce que le copolymère blocs A-B-A de la matrice est un copolymère polystyrène-polyéthylène-butylène-polystyrène.

4. Pansement selon l'une quelconque des revendications 1 ou 2, caractérisé en ce que le plastifiant de la matrice est une vaseline.

5. Pansement selon l'une quelconque des revendications 1 à 4, caractérisé en ce qu'il comprend dans sa matrice (i) 15 à 25 parties en poids de polystyrène-polyéthylène-butylène-polystyrène dont le rapport motifs styrène/motifs éthylène-butylène est inférieur à 0,5 et de préférence de l'ordre de 0,4, et (ii) 75 à 85 parties en poids de vaseline dont le point de goutte est compris entre 40 et 60°C.

6. Pansement selon l'une quelconque des revendications 1 à 5, caractérisé en ce qu'il comprend en outre dans sa matrice un principe actif en quantité thérapeutiquement efficace.

7. Pansement selon la revendication 6, caractérisé en ce que le principe actif est choisi parmi les anti-inflammatoires, les antalgiques, les antibiotiques, les antifongiques les antibactériens, les antiseptiques, les anesthésiques, les enzymes protéolytiques, les adjuvants de la cicatrisation, les facteurs de croissance et la propolis.

8. Pansement selon l'une quelconque des revendications 6 ou 7, caractérisé en ce qu'il comprend en outre dans sa matrice un agent promoteur du relargage du principe actif.

9. Pansement selon l'une quelconque des revendications 1 à 8, caractérisé en ce qu'il comprend en outre une trame noyée dans la masse constituée par le mélange dudit copolymère blocs A-B-A et dudit plastifiant.

10. Pansement selon l'une quelconque des revendications 1 à 9, caractérisé en ce qu'il comprend en outre un support solidaire de l'une des faces de la matrice.

11. Procédé de préparation d'un pansement selon la revendication 1 ou 2, caractérisé en ce qu'il comprend :
- le mélange de 10 à 30 parties en poids dudit copolymère blocs A-B-A et de 70 à 90 parties en poids dudit plastifiant,
- le malaxage du mélange résultant pendant 1 heure, puis
- l'enduction à une température inférieure de 10 à 40°C à la température de ramollissement dudit copolymère sur un support pelable avant emploi, de façon à obtenir une épaisseur dudit mélange comprise entre 10 µm et 1 cm.

## Revendications (Revendications pour l'(les) Etat(s) contractant(s) suivant(s): ES, GR)

1. Procédé de préparation d'un pansement à matrice, qui comprend dans sa matrice 10 à 30 parties en poids d'un copolymère blocs A-B-A où le bloc terminal A est du type polystyrène et le bloc central B est du type polyoléfine saturée, et 70 à 90 parties en poids de plastifiant, ledit procédé étant caractérisé en ce qu'il comprend :
- le mélange de 10 à 30 parties en poids dudit copolymère blocs A-B-A et de 70 à 90 parties en poids dudit plastifiant,
- le malaxage du mélange résultant pendant 1 heure, puis
- l'enduction à une température inférieure de 10 à 40°C à la température de ramollissement dudit copolymère sur un support pelable avant emploi, de façon à obtenir une épaisseur dudit mélange comprise entre 10 µm et 1 cm.

2. Procédé selon la revendication 1, caractérisé en ce que l'on mélange 10 à 30 parties en poids dudit copolymère blocs A-B-A et 90 à 70 parties en poids dudit plastifiant pour 100 parties en poids de ladite matrice.

3. Procédé selon la revendication 1 ou 2, caractérisé en ce que le copolymère blocs A-B-A de la matrice est un copolymère polystyrène-polyéthylène-butylènepolystyrène.

4. Procédé selon l'une quelconque des revendications 1 ou 2, caractérisé en ce que le plastifiant de la matrice est une vaseline.

5. Procédé selon l'une quelconque des revendications 1 à 4, caractérisé en ce que la matrice comprend (i) 15 à 25 parties en poids de polystyrène-polyéthylène-butylène-polystyrène dont le rapport motifs styrène/motifs éthylène-butylène est inférieur à 0,5 et de préférence de l'ordre de 0,4, et (ii) 75 à 85 parties en poids de vaseline dont le point de goutte est compris entre 40 et 60°C.

6. Procédé selon l'une quelconque des revendications 1 à 5, caractérisé en ce que la matrice comprend en outre un principe actif en quantité thérapeutiquement efficace.

7. Procédé selon la revendication 6, caractérisé en ce que le principe actif est choisi parmi les anti-inflammatoires, les antalgiques, les antibiotiques, les antifongiques, les antibactériens, les antiseptiques, les anesthésiques, les enzymes protéolytiques, les adjuvants de la cicatrisation, les facteurs de croissance et la propolis.

8. Procédé selon l'une quelconque des revendications 6 ou 7, caractérisé en ce que la matrice comprend en outre un agent promoteur du relargage du principe actif.

## Claims (Claims for the following Contracting State(s): AT, BE, CH, DE, DK, FR, GB, IT, LI, LU, MC, NL, PT, SE)

1. A dressing with a matrix containing a block copolymer A-B-A, said dressing being characterized in that it comprises in its matrix 10 to 30 parts by weight of a block copolymer wherein the terminal block A is of the polystyrene type and the central block B is of the saturated polyolefine type, and 70 to 90 parts by weight of plasticizer.

2. A dressing according to claim 1, characterized in that its matrix consists of 10 to 30 parts by weight of said block copolymer A-B-A and of 90 to 70 parts by weight of said plasticizer for 100 parts by weight of said matrix.

3. A dressing according to claim 1, wherein the block copolymer A-B-A of the matrix is a polystyrene-polyethylene-butylene-polystyrene copolymer.

4. A dressing according to claim 1 or claim 2, wherein the plasticizer of the matrix is a petrolatum.

5. A dressing according to any one of claims 1 to 4, which comprises in its matrix (i) 15 to 25 parts by weight of polystyrene-polyethylene-butylene-polystyrene in which the ratio styrene units/ethylene-butylene units is lower than 0.5 and preferably of the order of 0.4, and (ii) 75 to 85 parts by weight of petrolatum with a drop point of between 40 and 60°C.

6. A dressing according to any one of claims 1 to 5, which further comprises in its matrix a therapeutically effective amount of an active ingredient.

7. A dressing according to claim 6, wherein the active ingredient is selected from antiinflammatories, analgesics, antibiotics, antifungal agents, antibacterial agents, antiseptics, anesthetics, proteolytic enzymes, healing adjuvants, growth factors and propolis.

8. A dressing according to claim 6 or claim 7, which further comprises in its matrix an agent for promoting the release of the active ingredient.

9. A dressing according to any one of claims 1 to 8, which further comprises a web embedded in the mass consisting of the mixture of said block copolymer A-B-A and said plasticizer.

10. A dressing according to any one of claims 1 to 9, which further comprises a support firmly fixed to one face of the matrix.

11. A method for preparing a dressing according to claim 1 or 2, which comprises:
- mixing 10 to 30 parts by weight of said block copolymer A-B-A and 70 to 90 parts by weight of said plasticizer,
- malaxating the resulting mixture for 1 hour, then
- coating it, at a temperature 10 to 40°C below the softening point of said copolymer, on to a support, which can be peeled off before use, so as to give a thickness of said mixture of between 10 µm and 1 cm.

## Claims (Claims for the following Contracting State(s): ES, GR)

1. A method for preparing a dressing with a matrix which comprises in its matrix 10 to 30 parts by weight of a block copolymer A-B-A wherein the terminal block A is of the polystyrene type and the central block B is of the saturated polyolefine type, and 70 to 90 parts by weight of plasticizer, said method being characterized in that it comprises:
- mixing 10 to 30 parts by weight of said block copolymer A-B-A and 70 to 90 parts by weight of said plasticizer,
- malaxating the resulting mixture for 1 hour, then
- coating it, at a temperature 10 to 40°C below the softening point of said copolymer, on to a support, which can be peeled off before use, so as to give a thickness of said mixture of between 10 µm and 1 cm.

2. A method according to claim 1, wherein 10 to 30 parts by weight of said block copolymer A-B-A and 90 to 70 parts by weight of said plasticizer for 100 parts by weight of said matrix are mixed.

3. A method according to claim 1, wherein the block copolymer A-B-A of the matrix is a polystyrene-polyethylene-butylene-polystyrene copolymer.

4. A method according to claim 1 or claim 2, wherein the plasticizer of the matrix is a petrolatum.

5. A method according to any one of claims 1 to 4, wherein the matrix comprises (i) 15 to 25 parts by weight of polystyrene-polyethylene-butylene-polystyrene in which the ratio styrene units/ethylene-butylene units is lower than 0.5 and preferably of the order of 0.4, and (ii) 75 to 85 parts by weight of petrolatum with a drop point of between 40 and 60°C.

6. A method according to any one of claims 1 to 5, wherein the matrix further comprises a therapeutically effective amount of an active ingredient.

7. A method according to claim 6, wherein the active ingredient is selected from antiinflammatories, analgesics, antibiotics, antifungal agents, antibacterial agents, antiseptics, anesthetics, proteolytic enzymes, healing adjuvants, growth factors and propolis.

8. A method according to claim 6 or claim 7, wherein the matrix further comprises an agent for promoting the release of the active ingredient.

## Patentansprüche (Patentansprüche für folgende(n) Vertragsstaat(en): AT, BE CH, DE, DK, FR, GB, IT, LI, LU, MC, NL, PT, SE)

1. Verband mit einer Matrix, enthaltend ein Blockcopolymer A-B-A, dadurch gekennzeichnet, daß er in seiner Matrix 10 bis 30 Gewichtsteile eines Blockcopolymeren enthält, worin der terminale Block A vom Polystyrol-Typ ist und der mittlere Block B vom gesättigten Polyolefin-Typ ist, und 70 bis 90 Gewichtsteile Weichmacher.

2. Verband nach Anspruch 1, dadurch gekennzeichnet, daß seine Matrix aus 10 bis 30 Gewichtsteilen des Blockcopolymeren A-B-A und aus 90 bis 70 Gewichtsteilen des Weichmachers auf 100 Gewichtsteile der Matrix besteht.

3. Verband nach Anspruch 1 oder 2, dadurch gekennzeichnet, daß das Blockcopolymer A-B-A der Matrix ein Polystyrol-Polyethylen-Butylen-Polystyrol-Copolymer ist.

4. Verband nach einem der Ansprüche 1 oder 2, dadurch gekennzeichnet, daß der Weichmacher der Matrix eine Vaseline ist.

5. Verband nach einem der Ansprüche 1 bis 4, dadurch gekennzeichnet, daß er in seiner Matrix umfaßt (i) 15 bis 25 Gewichtsteile Polystyrol-Polyethylen-Butylen-Polystyrol, wovon die Styrol-Wiederholungseinheit/Ethylen-Butylen-Wiederholungseinheit unter 0,5 und vorzugsweise in der Größenordnung von 0,4 liegt, und (ii) 75 bis 85 Gewichtsteile Vaseline, deren Tropfpunkt zwischen 40 und 60 °C liegt.

6. Verband nach einem der Ansprüche 1 bis 5, dadurch gekennzeichnet, daß er unter anderem in seiner Matrix einen Wirkstoff in einer therapeutisch wirksamen Menge umfaßt.

7. Verband nach Anspruch 6, dadurch gekennzeichnet, daß der Wirkstoff ausgewählt ist aus Entzündungshemmern, Analgetika, Antibiotika, Fungiziden, Bakteriziden, Antiseptika, Anästhetika, proteolytischen Enzymen, Wundheilmitteln, Wachstumsfaktoren und Propolis.

8. Verband nach einem der Ansprüche 6 oder 7, dadurch gekennzeichnet, daß er unter anderem in seiner Matrix ein Mittel zur Beschleunigung der Freisetzung des Wirkstoffes umfaßt.

9. Verband nach einem der Ansprüche 1 bis 8, dadurch gekennzeichnet, daß er unter anderem einen Einschluß umfaßt, der in die Masse eingelassen ist, die aus der Mischung des Blockcopolymeren A-B-A und des Weichmachers besteht.

10. Verband nach einem der Ansprüche 1 bis 9, dadurch gekennzeichnet, daß er unter anderem einen mit einer der Matrixoberflächen verbundenen Träger umfaßt.

11. Verfahren zur Herstellung eines Verbandes nach Anspruch 1 oder 2, dadurch gekennzeichnet, daß es umfaßt:
- Mischen von 10 bis 30 Gewichtsteilen des Blockcopolymeren A-B-A und von 70 bis 90 Gewichtsteilen des Weichmachers,
- wobei das Verkneten der Mischung während 1 Stunde erfolgt, anschließend
- Beschichten bei einer Temperatur 10 bis 40 °C unterhalb der Erweichungstemperatur des Copolymeren auf einen vor dem Gebrauch abziehbaren Träger auf eine Weise, um eine Dicke der Mischung zwischen 10 µm und 1 cm zu erhalten.

## Patentansprüche (Patentansprüche für folgende(n) Vertragsstaat(en): ES, GR)

1. Verfahren zur Herstellung eines Matrixverbandes, der in seiner Matrix umfaßt 10 bis 30 Gewichtsteile eines Blockcopolymeren A-B-A, worin der terminale Block A vom Polystyrol-Typ ist und der mittlere Block B vom gesättigten Polyolefin-Typ ist, und 70 bis 90 Gewichtsteile Weichmacher, wobei das Verfahren dadurch gekennzeichnet ist, daß es umfaßt:
- Mischen von 10 bis 30 Gewichtsteilen des Blockcopolymeren A-B-A und von 70 bis 90 Gewichtsteilen des Weichmachers,
- wobei das Verkneten der Mischung während 1 Stunde erfolgt, anschließend
- Beschichten bei einer Temperatur 10 bis 40 °C unter der Erweichungstemperatur des Copolymeren auf einen vor dem Gebrauch abziehbaren Träger auf eine Weise, um eine Dicke der Mischung zwischen 10 µm und 1 cm zu erhalten.

2. Verfahren nach Anspruch 1, dadurch gekennzeichnet, daß 10 bis 30 Gewichtsteile des Blockcopolymeren A-B-A und 90 bis 70 Gewichtsteile des Weichmachers auf 100 Gewichtsteile der Matrix vermischt werden.

3. Verfahren nach Anspruch 1 oder 2, dadurch gekennzeichnet, daß das Blockcopolymer A-B-A der Matrix ein Polystyrol-Polyethylen-Butylen-Polystyrol-Copolymer ist.

4. Verfahren nach einem der Ansprüche 1 oder 2, dadurch gekennzeichnet, daß der Weichmacher der Matrix eine Vaseline ist.

5. Verfahren nach einem der Ansprüche 1 bis 4, dadurch gekennzeichnet, daß die Matrix umfaßt (i) 15 bis 25 Gewichtsteile Polystyrol-Polyethylen-Butylen-Polystyrol, wovon die Styrol-Wiederholungseinheit/Ethylen-Butylen-Wiederholungseinheit unter 0,5 und vorzugsweise in der Größenordnung von 0,4 liegt, und (ii) 75 bis 85 Gewichtsteile Vaseline, deren Tropfpunkt zwischen 40 und 60 °C liegt.

6. Verfahren nach einem der Ansprüche 1 bis 5, dadurch gekennzeichnet, daß die Matrix außerdem einen Wirkstoff in einer therapeutisch wirksamen Menge umfaßt.

7. Verfahren nach Anspruch 6, dadurch gekennzeichnet, daß der Wirkstoff ausgewählt wird aus Entzündungshemmern, Analgetika, Antibiotika, Fungiziden, Bakteriziden, Antiseptika, Anästhetika, proteolytischen Enzymen, Wundheilmitteln, Wachstumsfaktoren und Propolis.

8. Verfahren nach einem der Ansprüche 6 oder 7, dadurch gekennzeichnet, daß er unter anderem in seiner Matrix ein Mittel zur Beschleunigung der Freisetzung des Wirkstoffes umfaßt.
